# EUROPÄISCHE PATENTSCHRIFT

(11) **EP 1 477 469 B1**
(45) Veröffentlichungstag und Bekanntmachung des Hinweises auf die Patenterteilung: **27.07.2005**
(21) Anmeldenummer: 03010536.5
(22) Anmeldetag: 10.05.2003
(51) Int. Cl.: C07C 45/29, C07C 49/293

(54) **Salzfreies Verfahren zur Herstellung von Cyclobutanon**
Salt-free process for the production of cyclobutanone
Procédé sans sel pour la préparation de cyclobutanone

(43) Veröffentlichungstag der Anmeldung: 17.11.2004
(73) Patentinhaber: Degussa AG, 40474 Düsseldorf (DE)
(72) Erfinder: Bodmann, Kerstin, Dr., 45772 Marl (DE); Imig, Manuela, D-45768 Marl (DE); Köhler, Günther, Dr., 45770 Marl (DE); Brühl, Karl-Heinz, 46342 Velen (DE)

(56) Entgegenhaltungen:
- DE-A- 19 910 464
- MORI, KOHSUKE ET AL: "Controlled Synthesis of Hydroxyapatite-Supported Palladium Complexes as Highly Efficient Heterogeneous Catalysts" JOURNAL OF THE AMERICAN CHEMICAL SOCIETY (2002), 124(39), 11572-11573 , XP002255551
- JORNA, ANJA M. J. ET AL: "Heterogenization of a ruthenium catalyst on silica and its application in alcohol oxidation and stilbene epoxidation" REACTIVE & FUNCTIONAL POLYMERS (1996), 29(2), 101-114 , XP002255552
- DATABASE CROSSFIRE BEILSTEIN [Online] Beilstein Institut zur Förderung der Chemischen Wissenschaften, Frankfurt am Main, DE; Database accession no. Reaction ID 265578 XP002255553 & DOJARENKO, MARIE: CHEMISCHE BERICHTE, Bd. 1, Abt. B, 1927, Seiten 1536-1553, XP009018139 Berlin

## Beschreibung

Die Erfindung betrifft ein salzfreies Verfahren zur Herstellung von Cyclobutanon, bei dem Cyclopropylmethanol (Cyclopropylcarbinol) an einem heterogenen sauren Katalysator zu Cyclobutanol isomerisiert und dieses anschließend zu Cyclobutanon katalytisch dehydriert wird.

Cyclobutanon ist ein wichtiges und interessantes Zwischenprodukt in der organischen Chemie, inbesondere in der Wirkstoffsynthese zum Beispiel für Pharmawirkstoffe.
Die Herstellung von Cyclobutanon durch Umsetzung von Cyclopropylmethanol zu Cyclobutanol und anschließende Oxidation ist ein in der Literatur allgemein bekannter Syntheseweg (Org. Synth. 1981, 60, 20 - 25).
Die Isomerisierung von Cyclopropylmethanol zu Cyclobutanol in Gegenwart von verdünnter Salzsäure ist zum Beispiel in Org. Synth. 1986, 64, 50 - 56 beschrieben. Das Verfahren liefert nur in starker Verdünnung (Gew.-Verhältnis Cyclopropylmethanol : verdünnte Salzsäure = 1 : 12) das gewünschte Cyclobutanol. Zur Aufarbeitung wird die gesamte Säure neutralisiert und die wässrige Phase zusätzlich mit Natriumchlorid gesättigt und erst dann mit Diethylether extrahiert (Ausbeute 57 %). Aus technischer Sicht ist dieses Verfahren aufgrund des hohen Salzanfalls sowie der geringen Raum-Zeit-Ausbeute wenig vorteilhaft. Darüberhinaus werden bei Verwendung von Salzsäure chlorierte Nebenprodukte gebildet, die destillativ praktisch nicht abtrennbar sind (Can. J. Chem. 1980, 58 (11), 1075 - 1079) und im nachfolgenden Oxidationsschritt zu Korrosions- und Stabilitätsproblemen führen. Bei Verwendung von Salzsäure höherer Konzentration erhöht sich der Anteil an chlorierten Nebenprodukten. So wird zum Beispiel in US 5 905 176 die Herstellung von Cyclobutylchlorid durch Umsetzung von Cyclopropylmethanol mit 36 %iger Salzsäure im Temperaturbereich 35 - 120 °C beschrieben.

Die zur Oxidation von Cyclobutanol zu Cyclobutanon beschriebenen Verfahren verwenden die in der organischen Synthese üblichen Oxidationsmittel. Eine Zusammenstellung der bekannten Oxidationsmethoden ist in DE 199 10 464 gegeben. In der Regel handelt es sich dabei um Oxidationen in homogenen Systemen, aus denen das gewünschte Cyclobutanon erst durch Extraktion oder ähnliches isoliert werden muss. Diese Verfahren führen allgemein zu hohen Abfallmengen sowie zu hohen Belastungen durch toxische Reagenzien, wie zum Beispiel bei Verwendung von CrO₃/Oxalsäure laut Org. Synth. 1981, 60, 20 - 25. In diesem Verfahren ist die direkte Oxidation der aus der Isomerisierung von Cyclopropylmethanol erhaltenen wässrigen salzsauren Cyclobutanol-Lösung beschrieben. Für die Umsetzung von 49,5 g Cyclopropylmethanol (ergibt 14 - 16 g Cyclobutanon, Ausbeute 31 - 35 %) wird die Bildung von circa 1,2 l eines stark sauren, chromhaltigen Abwassers in Kauf genommen.

DE 199 10 464 beschreibt ebenfalls einen einstufigen diskontinuierlichen Prozess zur Herstellung von Cyclobutanon ausgehend von Cyclopropylmethanol, wobei das Cyclopropylmethanol zunächst mit verdünnter Salzsäure isomerisiert und dann die dabei erhaltene wässrige Lösung mit Chlorbleichlauge oxidiert wird. Das Verfahren führt aufgrund der benötigten großen Mengen verdünnter Salzsäure und Chlorbleichlauge ebenfalls zu einem erheblichen Salz- und Abwasseranfall (laut Beispiel 2 circa 1600 g Abwasser für 60 g Cyclobutanon). Außerdem führt die Durchführung der Oxidation mit Chlorbleichlauge im stark sauren Milieu zu einer verstärkten Bildung von Chlor, das aus der Reaktionsmischung ausgast. Die damit verbundenen sicherheitstechnischen und korrosionsbedingten Probleme sowie die verstärkte Bildung chlorierter Nebenprodukte machen das Verfahren aus technischer Sicht wenig attraktiv. Zudem wurde bei Lagertests mit nach der deutschen Patentanmeldung DE 199 10 464 hergestelltem Cyclobutanon innerhalb kurzer Zeit insbesondere bei stark chlorhaltigen Chargen eine merkliche Zersetzung sowie eine starke Verfärbung des Materials beobachtet.

Es bestand daher die Aufgabe, ein Verfahren zur Herstellung von Cyclobutanon ausgehend von Cyclopropylmethanol zu finden, das mit guter Raum-Zeit-Ausbeute sowie möglichst geringer Abfallbelastung durchzuführen ist. Außerdem war zudem eine möglichst einfache Maßstabsübertragung sowie die Möglichkeit einer kontinuierlichen Verfahrensführung wünschenswert.

Es wurde nun überraschend gefunden, dass sich in einem insgesamt salzfreien Verfahren durch Isomerisierung von Cyclopropylmethanol in Gegenwart eines heterogenen sauren Katalysators Cyclobutanol, das an einem heterogenen Katalysator zu Cyclobutanon dehydriert werden kann, gewinnen lässt. Gegenstand der Erfindung ist daher ein salzfreies Verfahren zur Herstellung von Cyclobutanon, wobei Cyclopropylmethanol in Gegenwart eines sauren heterogenen Katalysators zu Cyclobutanol isomerisiert und das Cyclobutanol anschließend an einem heterogenen Katalysator dehydriert wird.

Die Isomerisierung des Cyclopropylmethanols findet vorzugsweise in einem Lösemittel statt, wobei Wasser als Lösemittel besonders bevorzugt wird. Aus der wässrigen Mischung kann das Cyclobutanol ohne Zusatz von Salz mit gängigen Extraktionsmitteln isoliert werden. Das vorzugsweise nach destillativer Abtrennung des Extraktionsmittels erhaltene Cyclobutanol kann dann ohne weitere Reinigung und ohne Zusatz weiterer Reagenzien oder Lösemittel an einem heterogenen Katalysator zu Cyclobutanon dehydriert und der Reaktionsaustrag durch eine thermische Trennoperation, vorzugsweise eine Destillation, gegebenenfalls gereinigt werden.

Das Verfahren kann diskontinuierlich oder kontinuierlich durchgeführt werden.

Im Falle des Einsatzes einer wässrigen Mischung und bei Einsatz einer Extraktion fällt durch die Möglichkeit der Rückführung von Extraktionsmittel und wässriger Phase nach der extraktiven Aufarbeitung kaum Abwasser und kein Salz an. Abgesehen von bei der Reaktion gebildeten Nebenprodukten entstehen keine Abfälle durch Einsatz von Hilfsstoffen.

Überraschend wurde außerdem gefunden, dass bei der Isomerisierung in zum Beispiel wässrigem Medium selbst bei Minimierung des Wasseranteils in der Eduktmischung Cyclopropylmethanol/Wasser die Selektivität der Reaktion nicht zwangsläufig schlechter wird. Als positiver Effekt wurde in diesem Fall sogar eine erhebliche Verbesserung der Raum-Zeit-Ausbeute festgestellt.

Für die Isomerisierung werden heterogene saure Katalysatoren eingesetzt. Insbesondere können saure Ionenaustauscher-Harze und saure Tonmineralien verwendet werden. Als Beispiele für saure Ionenaustauscher-Harze seien vemetzte, sulfonierte Polystyrole wie zum Beispiel LEWATIT S100® der BAYER AG genannt. Als Beispiel für saure Tonmineralien seien saure Montmorillonite wie zum Beispiel KSF® und weitere K-Katalysatoren der Südchemie AG genannt.

Die Katalysatoren können entweder direkt als Suspension in einem Rührgefäß mit dem Cyclopropylmethanol in Kontakt gebracht werden oder bevorzugt bei einem kontinuierlichen Verfahren in ein zylindrisches, beheizbares Gefäß als Katalysatorbett, das vom Reaktionsmedium durchströmt wird (Festbett), eingebracht werden. Die kontinuierliche Ausführungsvariante kann als kontinuierlich betriebener Bypassreaktor eines Rührkessels oder als Reaktor im einmaligen beziehungsweise geraden Durchgang gefahren werden. Besonders bevorzugt ist die zuletzt erwähnte Vorgehensweise, da die kontinuierliche Verfahrensführung definierte Verweilzeiten erlaubt, die hohe Selektivitäten zur Folge haben.
Die Reaktion wird zweckmäßigerweise bei Temperaturen von 50 - 150 °C, vorzugsweise bei Temperaturen von 70 - 100 °C, insbesondere bei Temperaturen von 85 - 95 °C durchgeführt. Es ist zweckmäßig, das Katalysatorbett auf den bevorzugten Temperaturbereich vorzuheizen, wobei im Katalysatorbett beim Durchströmen der Zulaufmischung möglichst kein axialer Temperaturgradient auftreten soll. Für die erfindungsgemäße Ausführung zur Herstellung von Cyclobutanol ist ein schnelles Abkühlen des Produktstromes nach dem Verlassen der Reaktionszone von großer Bedeutung, um eine möglichst große Selektivität und Ausbeute zu erreichen.

Der heterogene saure Katalysator kann problemlos wiederverwendet werden.

Bei der Isomerisierung in einem wässrigen System kann das Verhältnis von Cyclopropylmethanol zu Wasser in sehr weiten Grenzen variiert werden. Aus wirtschaftlichen Gesichtspunkten und zur Optimierung der Raum-Zeit-Ausbeute ist es jedoch zweckmäßig, möglichst wenig Wasser in der Mischung anzuwenden. Geeignet ist daher ein Verhältnis Wasser zu Cyclopropylmethanol von 6 : 1 bis 0,5 : 1. Bevorzugt wird jedoch ein Verhältnis Wasser zu Cyclopropylmethanol von 3 : 1 bis 1,3 : 1 verwendet.

In der bevorzugten kontinuierlichen Fahrweise können (bezogen auf eingesetztes Cyclopropylmethanol) Katalysatorbelastungen (LHSV, liquid hourly space velocity) von 0,01 bis 2 1 Cyclopropylmethanol·h·⁻¹·l⁻¹ Kontakt bei optimaler Ausbeute und Selektivität realisiert werden. Die bevorzugte Katalysatorbelastung für den Prozess liegt bei 0,05 bis 1 l Cyclopropylmethanol·h·⁻¹·l⁻¹ Kontakt.

Nachdem das Eduktgemisch im Falle der Anwesenheit von Wasser als homogene Mischung aus Wasser und Cyclopropylmethanol der Reaktionszone zugeführt worden ist, bildet sich nach dem erfindungsgemäßen Verfahren Cyclobutanol, das jedoch nicht mit Wasser unbegrenzt mischbar ist und sich zum Teil als organische Phase vom Wasser abscheidet. Aus praktischen Gründen wird das zweiphasige Produktgemisch direkt in einer nachfolgenden Extraktionsstufe eingesetzt, da in der wässrigen Phase erhebliche Wertproduktmengen gelöst sind. Die wässrige Phase kann alternativ auch recyclisiert werden, indem nach Beaufschlagung mit neuem Cyclopropylmethanol diese Mischung erneut der Reaktionszone zugeführt wird.

Die Extraktion des Wertproduktes aus der Reaktionsmischung kann nach bekannten Methoden der Extraktion vorgenommen werden, wobei bevorzugt übliche Extraktionsmittel mit einem Siedepunkt, der deutlich vom Siedepunkt des Zielproduktes abweicht, anwendbar sind. Die Siedepunkte der verwendeten Extraktionsmittel liegen daher vorzugsweise < 100 °C oder > 130 °C. Als Beispiele seien Ether wie zum Beispiel Diethylether, Methyl-tert.-butylether, Butylethylether und Diisopropylether, Ester wie zum Beispiel Ethylacetat und Methylacetat, aliphatische und cycloaliphatische Kohlenwasserstoffe wie zum Beispiel Pentan, Hexan und Cyclohexan, aromatische Kohlenwasserstoffe wie zum Beispiel Benzol und Toluol und halogenierte Kohlenwasserstoffe wie zum Beispiel Dichlormethan und Chloroform, genannt. Die Verwendung von halogenierten Extraktionsmitteln ist jedoch weniger bevorzugt, da möglichst keine halogenierten Verbindungen mit dem Reaktionsmedium in Kontakt kommen sollen.
Aufgrund der vorteilhaften Dichteunterschiede des Wertprodukts zum Wasser ist es nicht notwendig, die wässrige Phase vor der Extraktion mit Salzen zu sättigen
Besonders bevorzugt ist die Extraktion mit Methyl-tert.-butylether (MTBE). Das nach Abtrennung des Extraktionsmittels anfallende Cyclobutanol-Rohprodukt enthält je nach Fahrweise 50 - 70 % Cyclobutanol, 5 - 10 % 3-Buten-1-ol sowie höhersiedende Nebenprodukte. Dieses Gemisch kann entweder destillativ aufgearbeitet oder bevorzugt direkt im nachfolgenden Dehydrierungsschritt eingesetzt werden. Durch Destillation des Rohprodukts kann Cyclobutanol in einer Reinheit von > 95 % erhalten und als solches verwendet werden. In der Praxis hat es sich jedoch bewährt, den Destillationsrückstand durch einmaliges Überdestillieren beispielsweise an einem Rotationsverdampfer oder an einer Kurzwegdestillationsapparatur von hochsiedenden Nebenkomponenten zu trennen. Auf diese Weise lassen sich Cyclobutanol-Reinheiten von 70 - 80 % erreichen.

Sowohl das Extraktionsmittel als auch die wässrige Phase sind problemlos wieder einsetzbar.

Die Dehydrierung von Cyclobutanol zu Cyclobutanon erfolgt im Allgemeinen an einem heterogenen Dehydrierungskatalysator, speziell an einem Kupfer und Chrom enthaltenen Katalysator. Der Katalysator kann Metalloxide als Promotoren enthalten. Bewährt hat sich insbesondere der Einsatz von Bariumoxid oder Manganoxid. Die Katalysatoren können trägerfrei oder zum Beispiel in Gegenwart von Aluminiumoxid oder Siliciumoxid als Trägermaterial verwendet werden.
Aufgrund der thermischen Instabilität von Cyclobutanol und Cyclobutanon sowie der Neigung von Cyclobutanon, unter oxidativen Bedingungen in einer Bayer-Villiger-Oxidation zu γ-Butyrolacton weiterzureagieren, ist eine erfolgreiche Umsetzung mit guten Selektivitäten und Ausbeuten bei den erforderlichen hohen Temperaturen überraschend. Auch der erfolgreich durchführbare Einsatz des im ersten Verfahrensschritt erhaltenen Produktgemisches ist, bei den gegebenen Reaktionsbedingungen (hohe Temperaturen, Gegenwart eines aktiven Katalysators) überraschend.

Die Dehydrierung wird vorzugsweise in einer Gasphasenreaktion durch Überleiten von zuvor verdampftem Cyclobutanol-Rohprodukt in einem Stickstoffstrom über ein Katalysatorfestbett in einem beheizten Ofen durchgeführt. Auch ein direktes Auftropfen des Cyclobutanol-Rohprodukts auf das Katalysatorbett ist möglich. Eine Verdünnung mit Lösemitteln ist nicht notwendig. Nach dem Austritt aus der Reaktionszone wird das Produktgemisch abgekühlt und vorzugsweise direkt der Destillation zugeführt. Eine vorherige Aufarbeitung ist nicht notwendig.

In einer alternativen Verfahrensführung wird der pulverisierte Katalysator in einem hochsiedenden Medium (zum Beispiel Weißöl) suspendiert und erhitzt. In die Katalysator-Suspension wird dann über ein Tauchrohr das Cyclobutanol-Rohprodukt eindosiert und das gebildete Produktgemisch über eine Destillationsbrücke abdestilliert. Dieses Sumpfphasen-Verfahren kann aus apparativen Gründen vorteilhaft sein, da es in einem normalen Rührkessel durchgeführt werden kann, ist aber hinsichtlich Umsatz und Raum-Zeit-Ausbeute gegenüber dem Gasphasen-Verfahren in der Regel unterlegen.
Der Katalysator kann direkt in der Reaktion eingesetzt werden. Eine Aktivierung zum Beispiel mit Wasserstoff ist nicht notwendig. Auch die Vermischung des Cyclobutanol-Rohproduktes mit Wasserstoff oder Wasserdampf zur Katalysatoraktivierung während der Reaktion ist nicht notwendig.
Der Katalysator kann wieder eingesetzt werden.

Die Gasphasen-Reaktion wird vorteilhaft bei einer Temperatur von 180 bis 350 °C durchgeführt. In einer bevorzugten Verfahrensführung wird die Dehydrierung in einem Temperaturbereich von 220 - 280 °C durchgeführt. Die Umsetzung wird ferner vorteilhaft bei Normaldruck oder geringem Überdruck bis vorzugsweise ca. 3 bar durchgeführt.

Bei der Gasphasendehydrierung können Katalysatorbelastungen (LHSV) im Bereich von 0,1 bis 5,0 l·h⁻¹·l⁻¹ Katalysator (bezogen auf Cyclobutanol-Rohprodukt flüssig) eingestellt werden. In einer bevorzugen Fahrweise liegt die Katalysatorbelastung im Bereich von 0,2 bis 4 l·h⁻¹·l⁻¹ Katalysator, in einer besonders bevorzugen Fahrweise liegt die Katalysatorbelastung im Bereich von 0,3 bis 3 l·h⁻¹·l⁻¹ Katalysator.

Das bei der Dehydrierung erhaltene Produktgemisch enthält, je nach Zusammensetzung des Einsatzstoffes, vorzugsweise ca. 60 - 65 % Cyclobutanon sowie je nach Vollständigkeit des Umsatzes 0 bis etwa 10 % Rest-Cyclobutanol. Die Zusammensetzung des Nebenproduktspektrums ändert sich nur geringfügig gegenüber der Zusammensetzung bezüglich der Nebenprodukte des Ausgangsmaterials. Das in der Isomerisierung als Nebenprodukt gebildete 3-Buten-1-ol wird mit hoher Selektivität zu Butyraldehyd umgesetzt, der destillativ gut abtrennbar ist. γ-Butyrolacton wird bei der Dehydrierung nur in geringfügigem Maße gebildet (< 2 %). Bei der Dehydrierung werden Ausbeuten bis zu 90 % bezogen auf eingesetztes Cyclobutanol erreicht.
Durch Destillation des Produktgemisches kann Cyclobutanon in einer Reinheit > 99 % erhalten werden.

Das Verfahren eignet sich grundsätzlich auch für die Herstellung alkylsubstituierter Cyclobutanone sowie für die Herstellung von cyclischen Ketonen mit größeren Ringen (zum Beispiel Cyclopentanon, Cyclohexanon).

Der Ausgangsstoff Cyclopropylmethanol ist zum Beispiel durch Hydrierung von Cyclopropancarbonsäuremethylester (DE-A-35 38 132) oder Cyclopropancarbaldehyd (EP-A-0 794 166) zugänglich.

### Beispiele

Die im Folgenden aufgeführten Beispiele sollen die Erfindung verdeutlichen aber nicht einschränken:

### Beispiel 1 (Kreislauffahrweise):

Eine Mischung aus 100 g (1,4 mol) Cyclopropylmethanol und 370 g entionisiertem Wasser wird aus einem Vorratsgefäß (unbeheizt) über eine Vorwärmzone (ca. 75 °C) von unten durch eine beheizbare 0,5 m-Kolonne, die mit 216 g LEWATIT S100® (H⁺-Form) und 190 g entionisiertem Wasser gefüllt ist, gepumpt (Geschwindigkeit ca. 300 ml/h). Die Temperatur innerhalb der Kolonne beträgt 90 - 95 °C. Nach Austritt aus der Kolonne wird die Reaktionsmischung wieder in den Vorratsbehälter zurückgeführt. Nach ca. 8 Stunden Reaktionszeit ist der Umsatz größer als 99 % und die Reaktion wird beendet. Das Reaktionsgemisch wird vollständig aus der Kolonne abgelassen und mit Methyl-tert-butylether (MTBE, ca. 360 g) in einem Perforator extrahiert. Nach Abtrennung von MTBE am Rotationsverdampfer werden 87 g Rotationsrückstand erhalten (Cyclobutanol-Gehalt: 70 %, Ausbeute: 62 %).

### Beispiel 2 (einmaliger Durchgang):

Eine Mischung von 100 g (1,4 mol) Cyclopropylmethanol und 280 g entionisiertem Wasser werden aus einem Vorratsgefäß durch die in Beispiel 1 beschriebene mit Ionentauscher gefüllte Kolonne (ohne vorherige Wasserzugabe) gepumpt (Geschwindigkeit ca. 200 ml/h). Das Produktgemisch wird am Kolonnenkopf abgenommen und direkt wie oben beschrieben der Extraktion zugeführt. Am Ende der Reaktion werden noch 150 g Wasser nachgepumpt, um die Kolonne zu spülen. Das Waschwasser wird ebenfalls zur Extraktion gegeben. Nach Abtrennung von MTBE werden 83 g Rückstand am Rotationsverdampfer erhalten (Cyclobutanol-Gehalt: 70 %, Ausbeute: 60 %).

### Beispiel 3 (einmaliger Durchgang):

Ein doppelwandiger, zylindrischer 9 l - Glasbehälter wird mit 6,5 kg LEWATIT S100® (H⁺-Form) befüllt und beidseitig durch Fritten verschlossen. Ein Hohlraum, der sich zwischem dem oberen Ende des Katalysatorbetts und dem oberen Ende des Glasbehälters ergibt, wird mit Glasperlen aufgefüllt, um das überschüssige Volumen zu verringern. Das Katalysatorbett wird von außen durch den Doppelmantel mit Hilfe einer Wärmeträgerflüssigkeit (Siliconöl) beheizt (Temperatur im Katalysatorbett 90 - 95°C). Eine Mischung aus 1,5 kg Cyclopropylmethanol (20,85 mol) und 4,2 kg Wasser wird mit einer Geschwindigkeit von 2 kg/h von unten durch das Katalysatorbett geleitet. Das Produktgemisch wird am oberen Ende des Reaktionsgefäßes abgenommen und mit MTBE extrahiert (4 x 3,6 kg). Die vereinigten organischen Phasen werden am Rotationsverdampfer eingeengt. Es werden 1,24 kg Roh-Cyclobutanol erhalten (Cyclobutanol-Gehalt: 63 %, Ausbeute: 51 %).

### Beispiel 4 (einmaliger Durchgang):

In der in Beispiel 3 beschriebenen Apparatur wird eine Mischung von 1,5 kg (20,85 mol) Cyclopropylmethanol und 2,1 kg Wasser umgesetzt (Pumpgeschwindigkeit: 2 kg/h). Die erhaltene Produktmischung wird mit MTBE extrahiert (3 x 3,0 kg) und die vereinigten organischen Phasen am Rotationsverdampfer eingeengt. Es werden 1,21 kg Roh-Cyclobutanol erhalten (Cyclobutanol-Gehalt: 60 %, Ausbeute: 49 %).

### Beispiel 5 (Sumpfphasendehydrierung):

Eine Suspension von 10 g Katalysator (Typ Cu-0202 P von Engelhard, enthält 67 Gew.-% Cu und 12 Gew.-% Cr) in 90 g Paraffinöl wird in einem 250 ml-Reaktionskolben mit Destillationsbrücke auf 160 °C erhitzt. In den Sumpf werden 108 g Cyclobutanol-Rohprodukt (Gehalt: 73 %, 1,14 mol) über einen Tropftrichter mit Einleitungsrohr dosiert und gleichzeitig über die Destillationsbrücke Destillat abgenommen. Die gesammelten Destillate werden per Gaschromatographie analysiert und dann erneut durch den Katalysatorsumpf geleitet. Nach vier Durchgängen werden 92 g Destillat erhalten mit einem mittleren Cyclobutanon-Gehalt von 73 % und einem Rest-Cyclobutanol-Gehalt von 13 % (Umsatz: 85 %, Selektivität > 98 %).

### Beispiel 6 (Gasphasendehydrierung):

Ein mit 50 g Katalysator (Typ H1044, Zusammensetzung ca. 27 g Kupferoxid, ca. 4 g Chromoxid, ca. 5 g Bariumoxid auf SiO₂) befülltes und zu beiden Enden mit Raschig-Ringen verschlossenes Quarzglasrohr wird in einen handelsüblichen, elektrisch beheizten Laborrohrofen eingebaut und die Temperatur in der Katalysatorzone auf 200 °C eingestellt. Über einen Vorverdampfer werden 30 g Cyclobutanol-Rohprodukt (Gehalt ca. 73 %, 0,3 mol) verdampft und dann über den Katalysator geleitet (LHSV = 0,18/h). Nach Austritt aus der Katalysatorzone wird das Reaktionsgemisch über einen Kühler abgekühlt und in einer Kühlfalle gesammelt. Es werden 27 g Reaktionsaustrag erhalten mit einer Zusammensetzung von 59 % Cyclobutanon und 19 % Cyclobutanol (Umsatz: 77 %, Selektivität: 96 %).

### Beispiel 7 (Gasphasendehydrierung):

In der in Beispiel 6 beschriebenen Versuchsapparatur (gleicher Katalysator) werden bei 250 °C 256 g Roh-Cyclobutanol (Gehalt ca. 74 %) umgesetzt (LHSV = 0,33/h). Es werden 243 g Reaktionsaustrag mit einer mittleren Zusammensetzung von 63 % Cyclobutanon und 12 % Cyclobutanol erhalten (Umsatz: 84 %, Selektivität > 98 %). Durch Destillation des Produktgemisches an einer 1 m Multifil-Kolonne werden 102 g Cyclobutanon mit einer Reinheit > 99 % (Sdp.: 97 - 99 °C, Destillationsausbeute: 67 %) erhalten.

### Beispiel 8 (Gasphasendehydrierung):

Das Quarzglasrohr wird mit 50 g Katalysator (Typ H1044 bei 700 °C calciniert) gefüllt und wie in Beispiel 7 in den Laborrohrofen eingebaut. Dann werden ebenfalls wie beschrieben 135 g (1,3 mol) Roh-Cyclobutanol (Gehalt 71 %) bei 250 °C über den Katalysator geleitet (LHSV = 0,33/h). Es werden 127 g Reaktionsaustrag mit einer mittleren Zusammensetzung von 58 % Cyclobutanon und 2,5 % Cyclobutanol erhalten (Umsatz: 97 %, Selektivität: 82 %).

### Beispiel 9 (Gasphasendehydrierung):

Das Quarzglasrohr wird mit 50 g Katalysator (Typ H1044 bei 650 °C calciniert) gefüllt und wie in Beispiel 7 beschrieben in den Laborrohrofen eingebaut. Dann werden 776 g Roh-Cyclobutanol (Gehalt: 71 %) bei 250 °C gasförmig über den Katalysator geleitet (LHSV = 0,33/h). Es werden 719 g Reaktionsaustrag mit einer mittleren Zusammensetzung von 67 % Cyclobutanon und 5 % Cyclobutanol erhalten (Umsatz: 94 %, Selektivität: 97 %). Destillation des Produktgemisches an einer 1 m Multifil-Kolonne ergibt 380 g Cyclobutanon (Reinheit > 95 %, Destillationsausbeute 81 %).

### Beispiel 10 (Gasphasendehydrierung):

Das Quarzglasrohr wird mit 50 g Katalysator (Typ H1044 bei 650 °C calciniert) gefüllt und wie in Beispiel 7 beschrieben in den Laborrohrofen eingebaut. Dann werden 876 g Roh-Cyclobutanol (Gehalt: 75 %) bei 250 °C gasförmig über den Katalysator geleitet (LHSV = 1,5 h⁻¹). Es werden 832 g Reaktionsaustrag mit einer mittleren Zusammensetzung von 67 % Cyclobutanon und 8 % Cyclobutanol erhalten (Umsatz: 90 %, Selektivität: 97 %). Destillation des Produktgemisches an einer 1 m Multifil-Kolonne ergibt 516 g Cyclobutanon (Reinheit > 95 %, Destillationsausbeute 80 %).

## Patentansprüche

1. Verfahren zur Herstellung von Cyclobutanon,
**dadurch gekennzeichnet,**
**dass** Cyclopropylmethanol in Gegenwart eines sauren heterogenen Katalysators zu Cyclobutanol isomerisiert und das Cyclobutanol anschließend an einem heterogenen Katalysator dehydriert wird.

2. Verfahren nach Anspruch 1,
**dadurch gekennzeichnet,**
**dass** das Cyclopropylmethanol als Mischung mit Wasser eingesetzt wird.

3. Verfahren nach Anspruch 2,
**dadurch gekennzeichnet,**
**dass** das Cyclobutanol aus der wässrigen Mischung extrahiert wird und das Extrakt mit oder ohne Lösemittel der Dehydrierung zugeführt wird.

4. Verfahren zur Herstellung von Cyclobutanon nach Anspruch 1,
**dadurch gekennzeichnet,**
**dass** der heterogene saure Katalysator als Festbett eingesetzt wird.

5. Verfahren zur Herstellung von Cyclobutanon nach Anspruch 1,
**dadurch gekennzeichnet,**
**dass** der heterogene saure Katalysator als Suspension eingesetzt wird.

6. Verfahren zur Herstellung von Cyclobutanon nach Anspruch 1,
**dadurch gekennzeichnet,**
**dass** der heterogene saure Katalysator ein saurer Ionenaustauscher ist.

7. Verfahren zur Herstellung von Cyclobutanon nach Anspruch 6,
**dadurch gekennzeichnet,**
**dass** der Ionenaustauscher ein vernetztes Polystyrol mit Sulfonsäuregruppen ist.

8. Verfahren zur Herstellung von Cyclobutanon nach Anspruch 7,
**dadurch gekennzeichnet,**
**dass** die Mischung aus Cyclopropylmethanol und Wasser im einmaligen Durchgang durch ein Bett von sauren Ionenaustauschern mit Sulfonsäuregruppen geleitet wird.

9. Verfahren zur Herstellung von Cyclobutanon nach Anspruch 1,
**dadurch gekennzeichnet,**
**dass** die Isomerisierung von Cyclopropylmethanol bei 50 - 150°C durchgeführt wird.

10. Verfahren zur Herstellung von Cyclobutanon nach Anspruch 1,
**dadurch gekennzeichnet,**
**dass** die Isomerisierung von Cyclopropylmethanol bei 70 - 100 °C durchgeführt wird.

11. Verfahren zur Herstellung von Cyclobutanon nach Anspruch 1,
**dadurch gekennzeichnet,**
**dass** die Katalysatorbelastung bei der Isomerisierung 0,01 bis 21 Cyclopropylmethanol·h⁻¹·l⁻¹ Katalysator beträgt.

12. Verfahren zur Herstellung von Cyclobutanon nach Anspruch 2,
**dadurch gekennzeichnet,**
**dass** das Verhältnis von Wasser zu Cyclopropylmethanol 6 : 1 bis 0,5 : 1 beträgt.

13. Verfahren nach zur Herstellung von Cyclobutanon nach Anspruch 2,
**dadurch gekennzeichnet,**
**dass** das Verhältnis von Wasser zu Cyclopropylmethanol 3 : 1 bis 1,3 : 1 beträgt.

14. Verfahren zur Herstellung von Cyclobutanon nach Anspruch 1,
**dadurch gekennzeichnet,**
**dass** die Dehydrierung in einer Gasphasenreaktion durch Überleiten von Cyclobutanol-Rohprodukt über den Katalysator in einmaligem Durchgang erfolgt und das erhaltene Cyclobutanon-Rohprodukt ohne weitere Aufarbeitung zur Destillation eingesetzt wird.

15. Verfahren zur Herstellung von Cyclobutanon nach Anspruch 1,
**dadurch gekennzeichnet,**
**dass** die Dehydrierung in einer Suspension in einem hochsiedenden Medium in Gegenwart eines pulverisierten Katalysators erfolgt.

16. Verfahren zur Herstellung von Cyclobutanon nach Anspruch 1,
**dadurch gekennzeichnet,**
**dass** der heterogene Dehydrierungskatalysator Kupfer und Chrom enthält.

17. Verfahren zur Herstellung von Cyclobutanon nach Anspruch 1,
**dadurch gekennzeichnet,**
**dass** die Dehydrierung in der Gasphase bei einer Temperatur von 180 - 350 °C durchgeführt wird.

18. Verfahren zur Herstellung von Cyclobutanon nach Anspruch 1,
**dadurch gekennzeichnet,**
**dass** die Dehydrierung in der Gasphase bei einer Temperatur von 220 - 280 °C durchgeführt wird.

19. Verfahren zur Herstellung von Cyclobutanon nach Anspruch 1,
**dadurch gekennzeichnet,**
**dass** bei der Dehydrierung Katalysatorbelastungen von 0,1 bis 5 l·h⁻¹·l⁻¹ Katalysator eingestellt werden.

## Claims

1. A process for preparing cyclobutanone,
**characterized in that**
cyclopropylmethanol is isomerized to cyclobutanol in the presence of an acidic heterogeneous catalyst and cyclobutanol is subsequently dehydrogenated over a heterogeneous catalyst.

2. A process according to claim 1,
**characterized in that**
the cyclopropylmethanol is used as a mixture with water.

3. A process according to claim 2,
**characterized in that**
the cyclobutanol is extracted from the aqueous mixture and the extract is fed to the dehydrogenation with or without solvent.

4. A process for preparing cyclobutanone according to claim 1,
**characterized in that**
the heterogeneous acidic catalyst is used as a fixed bed.

5. A process for preparing cyclobutanone according to claim 1,
**characterized in that**
the heterogeneous acidic catalyst is used as a suspension.

6. A process for preparing cyclobutanone according to claim 1,
**characterized in that**
the heterogeneous acidic catalyst is an acidic ion exchanger.

7. A process for preparing cyclobutanone according to claim 6,
**characterized in that**
the ion exchanger is a crosslinked polystyrene having sulfonic acid groups.

8. A process for preparing cyclobutanone according to claim 7,
**characterized in that**
the mixture of cyclopropylmethanol and water is passed in single pass through a bed of acidic ion exchangers having sulfonic acid groups.

9. A process for preparing cyclobutanone according to claim 1,
**characterized in that**
the isomerization of cyclopropylmethanol is carried out at 50-150°C.

10. A process for preparing cyclobutanone according to claim 1,
**characterized in that**
the isomerization of cyclopropylmethanol is carried out at 70-100°C.

11. A process for preparing cyclobutanone according to claim 1,
**characterized in that**
the liquid hourly space velocity in the isomerization is from 0.01 to 2 l of cyclopropylmethanol·h⁻¹·l⁻¹ of catalyst.

12. A process for preparing cyclobutanone according to claim 2,
**characterized in that**
the ratio of water to cyclopropylmethanol is from 6:1 to 0.5:1.

13. A process for preparing cyclobutanone according to claim 2,
**characterized in that**
the ratio of water to cyclopropylmethanol is from 3:1 to 1.3:1.

14. A process for preparing cyclobutanone according to claim 1,
**characterized in that**
the dehydrogenation is effected in a gas phase reaction by passing the crude cyclobutanol product over the catalyst in single pass and using the crude cyclobutanone product obtained for distillation without further workup.

15. A process for preparing cyclobutanone according to claim 1,
**characterized in that**
the dehydrogenation is effected in a suspension in a high-boiling medium in the presence of a pulverulent catalyst.

16. A process for preparing cyclobutanone according to claim 1,
**characterized in that**
the heterogeneous dehydrogenation catalyst comprises copper and chromium.

17. A process for preparing cyclobutanone according to claim 1,
**characterized in that**
the dehydrogenation is carried out in the gas phase at a temperature of 180-350°C.

18. A process for preparing cyclobutanone according to claim 1,
**characterized in that**
the dehydrogenation is carried out in the gas phase at a temperature of 220-280°C.

19. A process for preparing cyclobutanone according to claim 1,
**characterized in that**
the liquid hourly space velocities attained in the hydrogenation are from 0.1 to 5 l·h⁻¹·l⁻¹ of catalyst.

## Revendications

1. Procédé de fabrication de cyclobutanone,
**caractérisé en ce qu'**
on isomérise le cyclopropylméthanol en présence d'un catalyseur hétérogène acide en cyclobutanol et on déshydrogène ensuite le cyclobutanol sur un catalyseur hétérogène.

2. Procédé selon la revendication 1,
**caractérisé en ce qu'**
on utilise le cyclopropylméthanol en tant que mélange avec de l'eau.

3. Procédé selon la revendication 2,
**caractérisé en ce qu'**
on extrait le cyclobutanol du mélange aqueux et on fait déshydrogéner l'extrait avec ou sans solvant.

4. Procédé de fabrication de cyclobutanone selon la revendication 1,
**caractérisé en ce que**
le catalyseur acide hétérogène est utilisé comme lit fixe.

5. Procédé de fabrication de cyclobutanone selon la revendication 1,
**caractérisé en ce que**
le catalyseur acide hétérogène est utilisé comme suspension.

6. Procédé de fabrication de cyclobutanone selon la revendication 1,
**caractérisé en ce que**
le catalyseur acide hétérogène est un échangeur d'ions acide.

7. Procédé de fabrication de cyclobutanone selon la revendication 6,
**caractérisé en ce que**
l'échangeur d'ions est un polystyrène réticulé comportant des groupes acide sulfonique.

8. Procédé de fabrication de cyclobutanone selon la revendication 7,
**caractérisé en ce que**
le mélange de cyclopropylméthanol et d'eau passe, en un seul passage, par un lit d'échangeurs d'ions acides comportant des groupes acide sulfonique.

9. Procédé de fabrication de cyclobutanone selon la revendication 1,
**caractérisé en ce que**
l'isomérisation du cyclopropylméthanol est réalisée à une température de 50 à 150 °C.

10. Procédé de fabrication de cyclobutanone selon la revendication 1,
**caractérisé en ce que**
l'isomérisation du cyclopropylméthanol est réalisée à une température de 70 à 100 °C.

11. Procédé de fabrication de cyclobutanone selon la revendication 1,
**caractérisé en ce que**
la charge de catalyseur lors de l'isomérisation est de 0,01 à 2 litres de cyclopropylméthanol.h⁻¹.l⁻¹ de catalyseur.

12. Procédé de fabrication de cyclobutanone selon la revendication 2.
**caractérisé en ce que**
le rapport de l'eau au cyclopropylméthanol est de 6 : 1 à 0,5 : 1.

13. Procédé de fabrication de cyclobutanone selon la revendication 2,
**caractérisé en ce que**
le rapport de l'eau au cyclopropylméthanol est de 3 : 1 à 1,3 : 1.

14. Procédé de fabrication de cyclobutanone selon la revendication 1,
**caractérisé en ce que**
la déshydrogénation est réalisée lors d'une réaction en phase gazeuse en faisant passer le produit brut de cyclobutanol par un catalyseur en un seul passage et en mettant en oeuvre le produit brut de cyclobutanone en vue de la distillation sans autre préparation.

15. Procédé de fabrication de cyclobutanone selon la revendication 1,
**caractérisé en ce que**
la déshydrogénation est réalisée dans une suspension dans un mflleu à haut point d'ébullftion en présence d'un catalyseur pulvérulent.

16. Procédé de fabrication de cyclobutanone selon la revendication 1,
**caractérisé en ce que**
le catalyseur de déshydrogénation hétérogène contient du cuivre et du chrome.

17. Procédé de fabrication de cyclobutanone selon la revendication 1,
**caractérisé en ce que**
la déshydrogénation est réalisée dans la phase gazeuse à une température de 180 à 350 °C.

18. Procédé de fabrication de cyclobutanone selon la revendication 1,
**caractérisé en ce que**
la déshydrogénation est réalisée dans la phase gazeuse à une température de 200 à 280°C.

19. Procédé de fabrication de cyclobutanone selon la revendication 1,
**caractérisé en ce que**
lors de la déshydrogénation, on ajuste des charges du catalyseur de 0,1 à 5 litres.h⁻¹.l⁻¹.
